(19) Europäisches Patentamt
European Patent Office
Office européen des brevets

(11) **EP 4 710 839 A1**

(12) **EUROPEAN PATENT APPLICATION**

(43) Date of publication:
**18.03.2026 Bulletin 2026/12**

(21) Application number: **24306493.8**

(22) Date of filing: **12.09.2024**

(51) International Patent Classification (IPC):
**A61B 3/00** (2006.01)    **A61B 3/028** (2006.01)
**A61B 3/04** (2006.01)    **A61B 3/09** (2006.01)
**A61B 3/103** (2006.01)    **A61B 3/08** (2006.01)

(52) Cooperative Patent Classification (CPC):
**A61B 3/09; A61B 3/0025; A61B 3/028; A61B 3/04;
A61B 3/08; A61B 3/103**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Essilor International
94220 Charenton-Le-Pont (FR)**

(72) Inventors:
• **MARIN, Gildas
92160 ANTONY (FR)**
• **TATUR, Guillaume
77144 MONTEVRAIN (FR)**
• **CALIXTE, Laurent
92100 BOULOGNE BILLANCOURT (FR)**

(74) Representative: **Jacobacci Coralis Harle
32, rue de l'Arcade
75008 Paris (FR)**

(54) **SYSTEM AND METHOD FOR ASSESSING A SUITABILITY BETWEEN A CORRECTIVE OPTICAL DEVICE AND A SUBJECT**

(57)    The invention relates to a system for assessing a suitability between a corrective optical device and a subject, the system being configured to:
- determine an accommodative demand (AS) and a convergence demand (VS) to which the subject is submitted in a virtual optical system, the virtual optical system representing the subject looking at a target through the corrective optical device,
- calculate, using a predictive vision model of the subject, a response of the subject to the accommodative and convergence demand, the response comprising an accommodation value (AR), a convergence value (VR) and a visual performance value (VP), the predictive vision model taking into account interactions between an accommodation sub-model (100) of the predictive vision model, a convergence sub-model (200) of the predictive vision model and a visual performance sub-model (300) of the predictive vision model,
- assess the suitability based on at least one of the accommodation value, the convergence value and the visual performance value.

**Fig.4**

**Description**

TECHNICAL FIELD OF THE INVENTION

The disclosure relates to the domain of corrective optical devices.
The disclosure relates more specifically to a system for assessing a suitability between a corrective optical device and a subject.

BACKGROUND INFORMATION AND PRIOR ART

It has been proposed systems to evaluate the suitability of a given corrective optical device, comprising in particular corrective ophthalmic lenses, to a subject. Those systems are based on simulations of visual tests performed by the subject, virtually equipped with the corrective optical device. Those systems determine a response of the subject to the virtual visual tests in terms of binocular vision comfort or of visual performance.
However, those systems do not give highly reliable results as they consider either binocular vision comfort or visual performance.
So, there is a need for a new system considering at the same time binocular vision comfort and visual performance when assessing a suitability between a corrective optical device and a subject.

SUMMARY OF THE INVENTION

Therefore, one object of the disclosure is to provide a system for assessing a suitability between a corrective optical device and a subject, the corrective optical device being intended to be worn in front of at least one eye of the subject to improve a vision of the subject, the system being configured to:

- determine an accommodative demand and a convergence demand to which the subject is submitted in a virtual optical system, the virtual optical system representing the subject looking at a target through the corrective optical device,
- calculate, using a predictive vision model of the subject, a response of the subject to the accommodative demand and the convergence demand, the response comprising an accommodation value, a convergence value and a visual performance value, the predictive vision model taking into account interactions between an accommodation sub-model of the predictive vision model, a convergence sub-model of the predictive vision model and a visual performance sub-model of the predictive vision model,
- assess the suitability based on at least one of the accommodation value, the convergence value and the visual performance value.

Thanks to the system according to the disclosure, binocular vision comfort and visual performance are both taken into account when assessing the suitability, i.e. the fitness, of the corrective optical device to the subject.
Indeed, binocular vision comfort depends on accommodation and convergence.
In a remarkable manner, the predictive vision model of the disclosure takes into account these three components, namely accommodation, convergence, and visual performance, as well as interactions between them. The predictive vision model is thus able to reflect the physiological links between accommodation, convergence, and visual performance. For instance, it can reflect the fact that accommodation generates some convergence and, conversely, convergence generates some accommodation and also the fact that visual performance depends on accommodation and that, reciprocally, visual performance is implied in various parameters (such as depth of focus or Panum area) that impact accommodation and convergence.
As a consequence, the suitability of the corrective optical device to the subject is reliably assessed since the response of the subject, calculated in terms of accommodation, convergence and visual performance, is extensive and thus close to what the physiological response of the subject would be. The suitability of the corrective optical device to the subject can for instance be assessed by evaluating at the same time binocular vision comfort and visual performance.
Other advantageous and non-limiting features of the method according to the disclosure are:

- the interactions comprise using an output of one sub-model among the accommodation sub-model, the convergence sub-model and the visual performance sub-model as an input of one of the two other sub-models among the accommodation sub-model, the convergence sub-model and the visual performance sub-model;
- the predictive vision model is designed such that an output of the visual performance sub-model has a feedback effect on an internal parameter of the accommodation sub-model and/or of the convergence sub-model;
- the system is configured to evaluate a depth of focus of the subject based on the visual performance value and to modify an accommodative gain of the accommodation sub-model as a function of the depth of focus of the subject;

- the system is configured to determine a Panum area of the subject based on the visual performance value and to modify a convergence gain of the convergence sub-model as a function of the Panum area of the subject;
- the predictive vision model also takes into account at least one parameter relative to the virtual optical system among an illumination condition and a visual or structural feature of the target;
- the accommodative demand to which the subject is submitted in the virtual optical system is determined based on a distance between the eye of the subject and the target and on an optical power value of the corrective optical device;
- the convergence demand to which the subject is submitted in the virtual optical system is determined based on the distance between the eye of the subject and the target and on a prismatic deviation power value of the corrective optical device;
- the corrective optical device is characterized by its optical surfaces, and at least one of the accommodative demand to which the subject is submitted in the virtual optical system and the convergence demand to which the subject is submitted in the virtual optical system is determined by ray tracing based on the optical surfaces of the corrective optical device;
- the system is configured, before calculating the response of the subject, to personalize the predictive vision model based on at least one measured parameter related to the vision of the subject;
- the at least one measured parameter related to the vision of the subject is selected in a group consisting of: pupil size, prescription, maximum acuity, maximum objective or subjective amplitude of accommodation, punctum proximum of accommodation, punctum remotum of accommodation, maximum convergence or punctum proximum of convergence, accommodation at rest or dark focus or tonic accommodation, convergence at rest or dark vergence, targeted visual performance for far vision, targeted visual performance for near vision, interpupillary distance or phoria and fusional reserves for far and near distances or any other equivalent optometric measurements;
- assessing the suitability comprises comparing at least one of the accommodation value, the convergence value and the visual performance value to a threshold or to a numerical evaluation function;
- the system is configured to calculate, based on the virtual optical system and on at least one of the accommodation value, the convergence value and the visual performance value, at least one visual effort of the subject, and assessing the suitability comprises comparing the at least one visual effort of the subject to a threshold.

The disclosure also relates to a method for assessing a suitability between a corrective optical device and a subject, the corrective optical device being intended to be worn in front of at least one eye of the subject to improve its vision, the method comprising:

- determining a virtual optical system representing the subject looking at a target through the corrective optical device,
- determining an accommodative demand and a convergence demand to which the subject is submitted in the virtual optical system,
- calculating, using a predictive vision model of the subject, a response of the subject to the accommodative demand and the convergence demand, the response comprising an accommodation value, a convergence value and a visual performance value, the predictive vision model taking into account interactions between an accommodation sub-model of the predictive vision model, a convergence sub-model of the predictive vision model and a visual performance sub-model of the predictive vision model,
- assessing the suitability based on at least one of the accommodation value, the convergence value and the visual performance value.

The disclosure also relates to a method for determining a desired value of an optical feature of a corrective optical device, the corrective optical device being intended to be worn in front of at least one eye of the subject to improve a vision of the subject, the method comprising:

- selecting a test value of the optical feature of the corrective optical device,
- assessing, based on a method for assessing a suitability as described above, the suitability between the corrective optical device and the subject when the optical feature of the corrective optical device has the test value,
- determining, based on the suitability, the desired value of the optical feature of the corrective optical device as equal or not to the test value.

The disclosure also relates to a method for manufacturing a corrective optical device to be worn in front of at least one eye of the subject to improve a vision of the subject, the method comprising:

- determining, based on a method for determining a desired value as described above, the desired value of the optical feature of the corrective optical device,
- manufacturing the corrective optical device such that the corrective optical feature complies with the desired value.

The disclosure also relates to a computer program product, comprising instructions which, when the program is executed by a computer, cause the computer to carry out a method for assessing a suitability as described above.

DETAILED DESCRIPTION OF EXAMPLE(S)

The following description, enriched with joint drawings that should be taken as non-limitative examples, will help understand the disclosure and figure out how it can be realized.
On the appended drawings:

- figure 1 is a schematic representation of a system according to the disclosure,
- figure 2 is a schematic representation of a virtual optical system representing the subject looking at a target through the corrective optical device,
- figure 3 is a bloc diagram representing steps allowing to implement a method for assessing a suitability between a corrective optical device and a subject,
- figure 4 is a bloc diagram schematically representing a predictive vision model,
- figure 5 is a graphical representation of an accommodation measurement performed on the subject showing objective accommodation as a function of target proximity,
- figure 6 is a bloc diagram representing steps allowing to implement a method for determining a desired value of an optical feature of a corrective optical device.

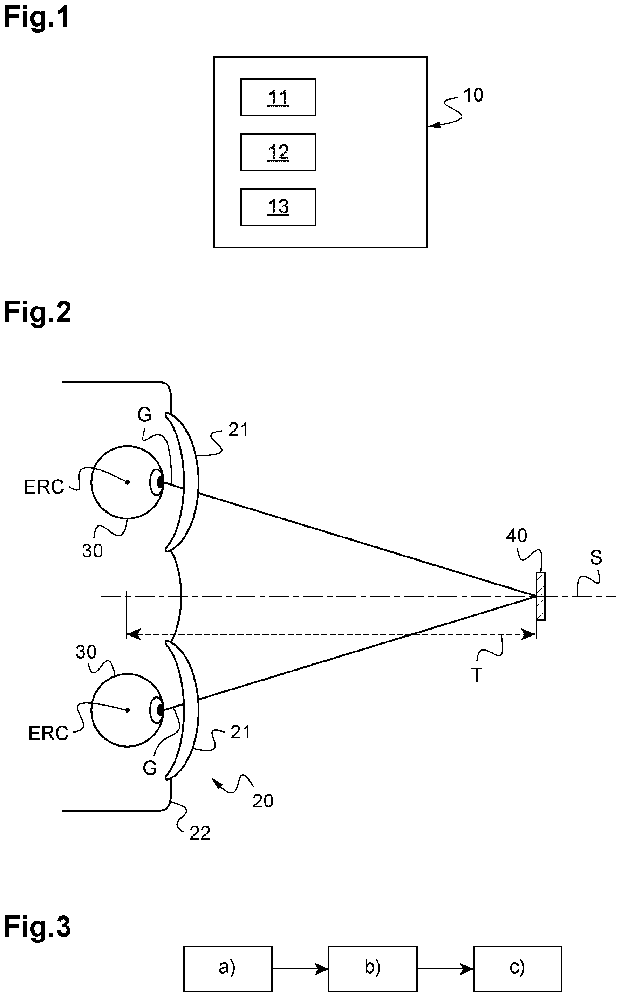

A system 10 for assessing a suitability between a corrective optical device 20 and a subject is represented in figure 1.
The corrective optical device 20, which can also be referred to as a corrective optical article, is intended to be worn in front of at least one eye of the subject. Put differently, the subject wears the corrective optical device 20. Here, as shown in figure 2, the corrective optical device 20 comprises two corrective ophthalmic lenses 21 mounted in a frame 22, each corrective ophthalmic lens 21 being positioned in front of one eye 30 of the subject. The corrective ophthalmic lenses 21 are for instance progressive ophthalmic lenses. As a variant, the corrective optical device may comprise only one corrective ophthalmic lens, or one or two ophthalmic contact lenses. The corrective optical device 20 may correspond to an already manufactured optical article or to an optical article to be manufactured.
The corrective optical device 20 is adapted to improve the vision of the subject by providing an optical correction to the subject, who may for example suffer from myopia, hypermetropia, astigmatism or presbyopia. The optical correction is associated to a corrective optical feature of the corrective optical device 20, here of its corrective ophthalmic lenses 21, such as addition, sphere, cylinder, axis of the cylinder, prism, axis of the prism, near field size, intermediate field size, far field size, progression length, or gradient of progression.
The suitability between the corrective optical device 20 and the subject is representative of how the corrective optical device 20 fits to the subject, that is to say how it responds to the subject's needs. The suitability is thus representative of a performance of the corrective optical device 20 with respect to the subject's vision.
The system 10 for assessing the suitability is a data-processing device configured, that is to say programmed, to implement a method for assessing the suitability between the corrective optical device 20 and the subject. As shown in figure 1, the system 10 comprises at least one processor 11 and at least one memory 12, which is here a non-transitory memory. The memory 12, which forms a computer-readable storage medium, comprises instructions which, when executed by the processor 11, allows implementing the method. The data stored in the memory 12 thus form a computer program product, comprising instructions which, when the program is executed by a computer, cause the computer to carry the method.
Here, the system 10 also comprises communication means 13, such as a user interface, which allows interacting with the system 10, for instance to store data on its memory 12 or to collect result data.
More specifically, the system 10 is configured to implement the following main steps represented in figure 3:

- a) determining an accommodative demand and a convergence demand to which the subject is submitted in a virtual optical system, the virtual optical system representing the subject looking at a target through the corrective optical device 20,
- b) calculating, using a predictive vision model of the subject, a response of the subject to the accommodative demand and the convergence demand, the response comprising an accommodation value, a convergence value and a visual performance value, the predictive vision model taking into account interactions between an accommodation sub-model of the predictive vision model, a convergence sub-model of the predictive vision model and a visual performance sub-model of the predictive vision model,
- c) assessing the suitability based on at least one of the accommodation value, the convergence value and the visual performance value.

The suitability may be expressed as an index, for instance having the value one when the corrective optical device 20 perfectly suits the subject, and having the value zero when it does not at all suit the subject. The suitability may also take discrete values (e.g. two basic literal values "comfortable" and "not comfortable", six values ranging from "not at all comfortable" to "very comfortable", etc.) or be a continuous numerical scale that is not necessarily linear. The suitability may for instance be determined as a value within a predetermined range, for instance between 0% and 100%, to more finely reflects the fitness of the corrective optical device 20 to the subject.

The suitability may also be calculated based on the accommodation value, the convergence value and the visual performance value, for instance as a weighted sum of those three values. As detailed later, such a weighted sum can thus be used in a comparison process or an optimization process, typically through a cost function, to find the corrective optical device which best suits the subject.

Step a)

In step a), the system 10 has access to numerical data representing the virtual optical system, including a virtual optical environment. The system 10 may retrieve those data from its memory 12 or may acquire them from its communication means 13.

The virtual optical system is representative, in a computing environment, of a visual task performed by the subject wearing the corrective optical device 20. The visual task for instance corresponds to a day-life situation, such as reading.

In the example illustrated in figure 2, the visual task consists in looking at a target 40, here with both eyes 30. During this visual task the subject is virtually equipped with the corrective optical device 20, which means here that the ophthalmic lenses 21 are placed in front of his eyes 30. Of course, more complex visual tasks may be defined in the virtual optical system, such as visual tasks involving moving targets.

Put differently, the virtual optical system is here a numerical representation, in a common frame of reference, of the subject looking at the target 40 through the corrective optical device 20.

The above-mentioned numerical data are at least representative of a position of the target 40 with respect to the eyes 30 of the subject.

The position of the target 40 may be defined by coordinates, such as 3D-coordinates, in the frame of reference. The frame of reference is for example linked to the eyes of the subject. The origin of the frame of reference is for instance the middle of a segment between the center of rotation of one of the two eyes and the center of rotation of the other of the two eyes.

According to the embodiment represented in figure 2, the position of the target 40 with respect to the eyes 30 of the subject is defined by a target distance T, i.e. a distance between the eyes 30 and the target 40. Here, the target distance T is expressed as a proximity value in diopters (the proximity corresponding to the inverse of the target distance). The target distance T may correspond to a distance between one eye 30 (whose position is for instance assimilated to the eye rotation center ERC) and the target 40 projected on a vertical symmetrical plane S of the head of the subject.

The above-mentioned numerical data are also representative of the corrective optical device 20. They for instance comprise values of optical features of the corrective optical device 2. Here, they comprise an optical power value and a prismatic deviation power value for each ophthalmic lens 21 (in diopters). The optical power value and the prismatic deviation power value may vary according to the portion of the ophthalmic lens 21 located in the field of view of the subject, that is to say the points of the lens intersecting the gaze directions. Classically, the prismatic deviation power value reflects the amount of lateral displacement of the target, placed at a given longitudinal distance, as seen through the ophthalmic lenses.

The target 40 may be further characterized in the virtual optical system. For instance, the above-mentioned numerical data may be representative of the following features of the target 40: shape, size, contrast, spatial frequency. By spatial frequency, it is meant the minimum angle of resolution of the smallest details to be seen (detected and analyzed) during the visual task.

Environmental conditions may also be further defined in the virtual optical system. For instance, the above-mentioned numerical data may be representative of the following features associated with conditions in which the virtual visual task takes place: illumination conditions, in particular luminance (also known as brightness), time of observation.

In step a), the system 10 is more specifically programmed to compute the accommodative demand and the convergence demand.

The subject is submitted to these demands in the sense that, in the virtual optical system, these demands are visually presented to him. These demands may thus be understood as an accommodative variation and a convergence variation that the eyes 30 of the subject need to perform so that the target 40 is accurately perceived by the subject. In the field of ophthalmology, these demands are sometimes referred to as stimuli, in particular in the sense that they trigger a response of the eyes 30. More specifically, the accommodative demand here corresponds to a proximity of the target seen through the corrective ophthalmic lenses 21 and the convergence demand to a proximity of gaze crossing (the gaze direction being referenced G in figure 2) in a plan comprising the eyes and a target fixation point (such as the plane of the figure 2). Alternatively, the convergence demand may be expressed as the angle or the prismatic deviation between the two gaze directions (one for each eye) needed to cross at the target. The gaze directions may not be horizontal, depending on the visual task. They may typically be oriented downward in near vision when reading or when walking. The deviation component that is parallel to the eye axis, inducing convergence, is commonly called horizontal deviation. Alternatively, vertical deviation may refer to the deviation component perpendicular to the gaze plane (i.e. the vertical deviation component for an horizontal gaze plane).

For each eye 30, the accommodative demand AS may be computed according to the following formula: AS = 1 / T - OP, wherein T is the target distance and OP the optical power value of the ophthalmic lens 21 in front of said eye 30. The accommodative demand is for instance comprised between -1 D and +5 D (D for diopters), typically between 0 and +4D. The sign of the accommodative demand is conventional, in this divulgation, positive values are used for closer targets. The accommodation demand may be negative for a non-corrected myopes. A negative demand may also mean that the subject may not be able to accommodate on the target. A zero value for the accommodation demand usually refers to infinity for an emmetrope or corrected subject in far vision.

For binocular vision, the accommodative demand may be given by the following formula: AS = [(1+d)ASright+(1-d)ASleft]/2,

with ASright the accommodative demand for the right eye, ASleft the accommodative demand for the left eye, and d = 0 if there is no dominance, 1>d>0 if the right eye is dominant and 0>d>-1 if the left eye is dominant.

For each eye 30, the convergence demand VS may be computed according to the following formula: VS = 1 / T - DP/PD, wherein T is the target distance, DP the difference in prismatic deviation power values of each ophthalmic lens 21 in front of said each eyes 30, expressed in prismatic diopters (1 prismatic diopter being equal to a deviation of 1 cm at 1 m), and PD the pupillary distance between the two eyes (in cm). The convergence demand is for instance comprised between -1 D and +5 D (D for diopters), typically between 0 and +4D.

When the above-mentioned numerical data comprise a numerical representation of the optical surfaces of the corrective optical device 20, for instance a 2D or a 3D representation of the optical surfaces of the ophthalmic lenses 21, the accommodative demand and/or the convergence demand may be computed by ray tracing based on the optical surfaces of the corrective optical device 20. The optical properties of the material of the corrective optical device (refractive index and eventually its variation with wavelength) may also be taken into account. Indeed, knowing the shape, and preferentially the material optical properties, of the ophthalmic lenses allows to precisely determine the gaze directions G of the eyes for a given position of the target, by applying the refraction laws through the complete path of light, and thus the accommodative demand and convergence demands.

Step b)

In step b), the system 10 computes, using the predictive vision model of the subject, a response of the subject to the accommodative demand and the convergence demand.

In other words, the response of the subject is a computed output of the predictive vision model when the accommodative demand and the convergence demand are used as inputs of the predictive vision model. The predictive vision model is thus "predictive" in the sense that it allows evaluating (here numerically by means of the accommodation value, the convergence value and the visual performance value) how the subject would react to the visual task. Using the predictive vision model is thus a data processing evaluating variations of optical features of the eyes of the subject (in particular accommodation, convergence, and visual performance) given the accommodative demand and the convergence demand. The predictive vision model is "of the subject" in the sense that it describes the vision of the subject.

The predictive vision model is preferentially personalized to the subject. This personalization means that internal parameters of the predictive vision model, that is to say variables of the predictive vision model (by opposition to the demands which are inputs of the model), may depend on the parameters relating to the subject.

This personalization may be relatively basic, for instance by making internal parameters of the predictive vision model dependent on easily accessible parameters relative to the subject, such as the age of the subject (see for example the parameter ACA below). Advantageously, the dependency of one or several parameters of the predictive vision model on age allows for example to take into account that accommodating is more restricted with age due to presbyopia.

This personalization may also be more advanced, for instance when the vision model takes into account one or more of the following parameters relative to the subject: pupil size, prescription, maximum acuity, maximum objective or subjective amplitude of accommodation, punctum proximum of accommodation, punctum remotum of accommodation, maximum convergence or punctum proximum of convergence, accommodation at rest or dark focus or tonic accommodation, convergence at rest or dark vergence, targeted visual performance for far vision, targeted visual performance for near vision, interpupillary distance or phoria and fusional reserves for far and near distances or any other equivalent optometric measurements. As described afterwards, the values of those parameters may be estimated, typically based on statistical models, or may be directly measured on the subject.

The predictive vision model is here used as a binocular vision model. However, the predictive vision model may easily be used as a monocular vision model by adjusting some of its internal parameters, for instance by setting gains of the convergence sub-model to zero.

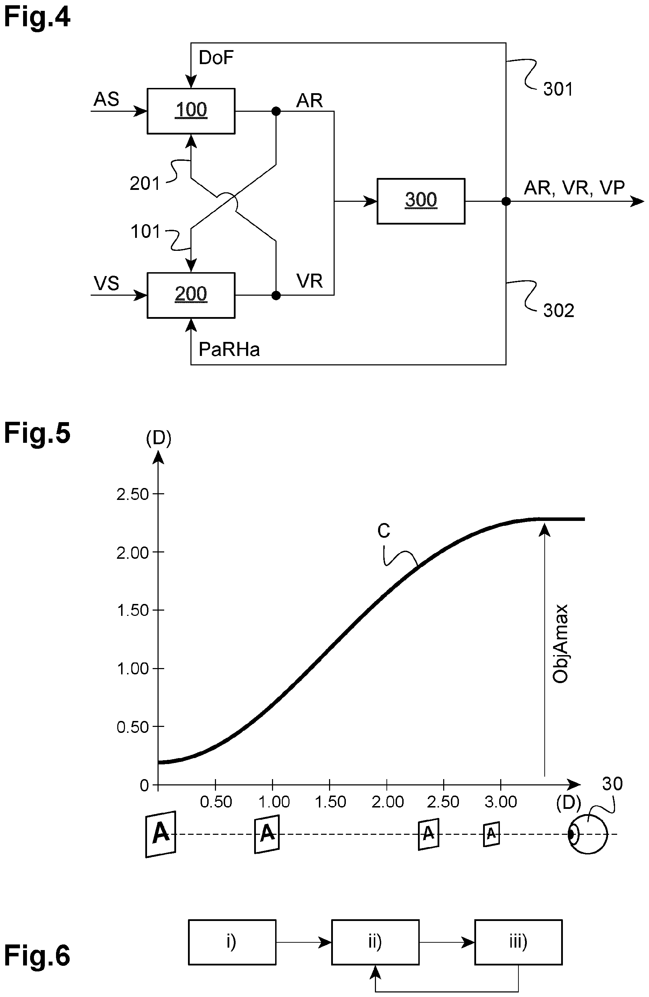

As represented in figure 4, the predictive vision model more specifically comprises the accommodation sub-model 100 giving the accommodation value, the convergence sub-model 200 giving the convergence value and the visual performance sub-model 300 giving the visual performance value. The accommodation value and the convergence value are here expressed in diopters, but any other well-known units may be used as above-mentioned for convergence. The visual performance value may correspond to a visual acuity which is for instance expressed in arc minutes, as a decimal acuity (expressed as a score out of six, ten or twenty), or in logMAR. The visual performance value may also correspond to a contrast sensitivity or a blur sensitivity or a subjective image quality or a reading speed or a depth perception or a stereoacuity or an ocular visual comfort. The ocular visual comfort may be expressed as describes in the following document "The zone of comfort: Predicting visual discomfort with stereo displays" Shibata, T., Kim, J., Hoffman, D. M., & Banks, M. S. (2011), Journal of Vision, 11(8):11, 1-29.

Regarding figure 4, attention is drawn to the fact that this figure is only a graphical representation of a mathematical object. This graphical representation is used for comprehension purposes. In other words, the graphical representation of figure 4 does not directly represent the programming of the system 10. As an example, showing the sub-models in a sequential order is merely illustrative since, in practice, the sub-models correspond to formulas (i.e. equations) encoded and stored on the memory 12 in no specific order.

Indeed, as detailed below, the predictive vision model is formed by a set of formulas giving the accommodation value, the convergence value and the visual performance value as a function of the accommodative demand and the convergence demand. Here, the predictive model is stored in the memory 12 under the form of computer-readable data.

The accommodation sub-model 100 more specifically refers to the formula giving the accommodation value (formula [1] below). The convergence sub-model 200 more specifically refers to the formula giving the convergence value (formula [2] below). The visual performance sub-model 300 more specifically refers to the formula giving the visual performance value (formula [3] below).

Nevertheless, figure 4 illustrates the following properties of the vision prediction model.

At least one output of one of the three sub-models is used as an input of one of the two other sub-models. The term "used as an input" means that the output is used to modify an internal parameter of the sub-model.

In particular, in figure 4, the branch referenced 101 illustrates a feedback effect of an output of the accommodation sub-model 100 on the convergence sub-model 200, which corresponds to the fact (illustrated by AR depending on VS in formula [1] below) that accommodation generates convergence.

The branch referenced 201 illustrates a feedback effect of an output of the convergence sub-model 200 on the accommodation sub-model 100, which corresponds to the fact (illustrated by VR depending on AS in formula [2] below) that convergence generates accommodation.

The branches referenced 301 and 302 illustrate a feedback effect of an output of the visual performance sub-model 300 on the accommodation sub-model 100 and/or the convergence sub-model 200, which corresponds to the fact that visual performance is implied in various parameters that impact accommodation and/or convergence, such as depth of focus or Panum area. Here, the system 10 is in particular configured to evaluate a depth of focus of the subject (referenced DoF below), that is to say to a value representative of the depth of focus, of the subject based on the visual performance value and to modify an accommodative gain (referenced AG below) of the accommodation sub-model 100 as a function of the depth of focus of the subject. Here, the system 10 is also configured to determine a Panum area of the subject (in this divulgation the horizontal radius of the Panum area referenced PaRHor below), that is to say a value representative of the Panum area, based on the visual performance value and to modify a convergence gain (referenced VG below) of the convergence sub-model 200 as a function of the Panum area of the subject. Indeed, the Panum area is proportional to the maximum perceived spatial frequency, which is linked to the visual performance value. The Panum area of the subject may be defined as the acceptable limits of fusion of binocular vision.

At this stage of the disclosure, a possible embodiment of the vision prediction model is described. Hereinafter, this vision prediction model is theoretically separated in two models, an accommodation-convergence model and a visual performance model.

First, the following parameters are introduced:

- AS is the accommodative demand,
- VS is the convergence demand,
- AR is the accommodation value,
- VR is the convergence value,
- dkF is the dark focus or accommodation at rest,
- dkV is the dark vergence or convergence at rest,
- AG is the accommodative gain,
- VG is the convergence gain,

- PRA is the punctum remotum of accommodation (objective ametropia), which is null in case the subject is emmetrope,
- PPA is the punctum proximum of accommodation,
- PPC is the punctum proximum of convergence,
- PRC is the punctum remotum of convergence,
- PaRHor is the horizontal Panum radius,
- DoF is the depth of focus,
- dPhAS is the dissociated phoria at a given accommodative demand, typically near vision about 40 cm (2.5 D proximity),
- dPhRx is the dissociated phoria at distance,
- Rx is the spherical equivalent that would be prescribed for far vision obtained from a subjective refraction,
- dPhPRA is the dissociated phoria for the punctum remotum of accommodation
- $\Delta$fr is the fusional reserve for far vision,
- ObjAA is the monocular objective amplitude of accommodation.
- ObjAmax is the maximal amplitude of objective accommodation,
- SF is the spatial frequency,
- Ecc is the retinal eccentricity,
- age is the age of the subject
- PD is the pupillary distance (in m).

The depth of focus (DoF) corresponds to the maximum change in target distances for which the visual performance loss is below a predefined threshold, the predefined threshold value may vary depending on the visual task, the target and an effective visual performance. For instance, when the vision of the subject is already blurred and thus the effective visual performance decreased, the depth of focus may be increased (more tolerance to blur) by lowering the predefined threshold.

For ObjAA and ObjAmax, both eyes may have the same values. Alternatively an average of both eyes or a weighted sum with dominance may be used. Different values may also be set for each eye. The following formula may also be used: ObjAA = 7.083/(1+EXP(0.2031*(age-36.2-0.6109))) given by the document "Minus lens stimulated accommodative lag as a function of age". Anderson, H. A., Glasser, A., Stuebing, K. K., & Manny, R. E. (2009). Optometry and Vision Science, 86(6), 685-694.

The accommodation-convergence model may be expressed as follows.

The accommodation value AR may be given by the following formula: AR = min(max(PRA, ARC), PPA) [1], where:

$$AR0 = [(AG-CAC.ACA)AS+(1-AG)(dkF+CAC(VS-dkV))]/(1-ACA.CAC)$$

The convergence value may be given by the following formula: VR = min(max(PRC, VR0), PPC) [2], where:

$$VR0 = [(VG-CAC.ACA)VS+(1-VG)(dkV+ACA(AS-dkF))]/(1-ACA.CAC)$$

However, if |VS-VR0|>PaRHor, VR = dkV and AR = dkF.

In the above formulas:

- ACA = (VS+dPhAS-dPhRx)/AS or ACA = 1-0.005age, where dPhAS+VS = dkV+ACA(AS-dkF) and dPhRx = dkV+ACA(Rx-dkF)-Rx,

$$CAC = 1/(0.5+ACA), \text{ or } CAC = max(0.24-0.0045age;0)*PD,$$

$$VG = \Delta fr/(\Delta fr+2PaRHor).(1-ACA.CAC)+ACA.CAC,$$

$$dkF = ObjAmax/3+PRA,$$

$$dkV = ACA.dkF+dPhPRA \text{ or } dkV = ACA.dkF \text{ with } dPhPRA = 0,$$

$$\Delta fr = 42.5 \text{ D},$$

$$PPA = ObjAA+PRA,$$

- PaRHor = 6+SF/4+3.75Ecc (in minutes of arc), PaRHor in diopters is equal to the product of PaRHor in minutes of arc multiplied by $\pi/(60*180*PD)$ where PD is the pupillary distance in meters,

$$AG = ObjAA/(ObjAA+DoF) \text{ or } AG = (ObjAA - DoF)/Obj\,AA$$

As a variant, the accommodation-convergence model may be given by the formulas below that are symmetrical between accommodation and convergence, and between remotum and proximum:

$$AR=AG.AS+(1-AG)AX,$$

$$VR=VG.VS+(1-VG)VX,$$

$$AX=dkF+CAC(VS-dkV),$$

$$VX=dkV+ACA(AS-dkF),$$

$$AG = 1-2.DoF/(PPA-PRA),$$

$$VG = 1-2.PaRHor/(PPC-PRC)$$

The accommodation-convergence model may also correspond to the model disclosed in the document "Quantitative Analysis of the Accommodative Convergence to Accommodation Ratio: Linear and Nonlinear Static Models", George K. Hung, 1997, IEEE TRANSACTIONS ON BIOMEDICAL ENGINEERING, VOL. 44, NO. 4. The accommodation-convergence model above-described has also similarities with the one described in document EP4335353.

Nevertheless, in a remarkable manner, the accommodation-convergence model above-described benefits in particular from a feedback effect of the visual performance value on the accommodation value and the convergence value by means of the variable accommodation and convergence gains ("variable" in the sense that they depend on the visual performance value, by opposition to fixed values).

The visual performance model may be expressed as follows, wherein VP is the visual performance value.

$$VP^2 = MAR0^2 + (K \cdot D\_pup \cdot \delta)^2 \ [3],$$

where:

- $\delta^2 = \Delta P^2 + k.A^2$ with $k=\frac{1}{2}$ or $\frac{1}{4}$ ($\delta$ in D),
- $\Delta P$ is the power error,
- A is the residual astigmatism,
- D_pup is the pupil diameter (in mm),
- K=0.41,
- $MAR0^2 = MARretina^2 + MARAb^2$,
- $MARAb = 0.897/D\_pup + 0.49 \ .(1-1/(1+0.017 \cdot D\_pup\ 3))-0.45$,
- $MARretina^2 = [1/(1.5-(age-35)/60)]^2 + MAREcc^2$,
- $MAREcc^2 = MAREcch^2 + MAREccv^2$,
- $MAREcch = 0.0075 \ Ecch^2 + 0.23 \ Ecch$,
- $MAREccv = 0.013 \ Eccv^2 + 0.33 \ Eccv$,
- Ecch and Eccv are horizontal and vertical eccentricity (in degrees) of the object position from the fixation point.

The visual performance model may also correspond to the visual acuity model disclosed in the documents:

- "Influence of combined power error and astigmatism on visual acuity", C. Fauquier, T. Bonnin, C. Miege, and E.

Roland, in Vision Science and its Applications, Technical Digest Series (Optica Publishing Group, 1995), paper SaE6,
- *"Phenomenological model of visual acuity"* Gómez-Pedrero JA, Alonso J.. J Biomed Opt. 2016 Dec 1;21 (12), or
- *"castleCSF - A contrast sensitivity function of color, area, spatiotemporal frequency, luminance and eccentricity"*, Ashraf, M., Mantiuk, R. K., Chapiro, A., & Wuerger, S. (2024). Castle CSF, Journal of Vision, 24(4):5, 1-38.

The dependency of the depth of focus (DoF) and the Panum Area (PaRHor) on visual performance, here on the visual acuity (VA), may be expressed by the following formulas:

$$VA(\delta+DoF^+)-VA(\delta)=TOL,$$

$$VA(\delta-DoF^-)-VA(\delta)=-TOL,$$

$$VA(x) = Log(MARx),$$

$$DoF=(DoF^+ + DoF^-)/2,$$

- TOL=max(0.05,abs[min(0.2,Log(MARobj/MARperceived))]),

where $DoF^+$ is the further limit of the depth of focus and $DoF^-$ is the closer limit of the depth of focus. Alternatively, DoF=sqrt([(KD_pup)$^2$.$\delta^2$+MAR0$^2$]10^ (2TOL)-MAR0$^2$)/(KD_pup)-$\delta$ PaRHor = 6+MARB/2+3.75Ecc MARB=max(MAROD, MAROS, MARobj), wherein MARobj is the minimum angle of resolution associated to the smallest details in the object to be detected, MARperceived is the minimum angle of resolution perceived (inverse of decimal visual acuity), MAROD is the minimum angle of resolution for the right eye and MAROS is the minimum angle of resolution for the left eye.

Advantageously, the predictive vision model may also take into account one or more parameters represented in the virtual optical system.
The predictive vision model may for instance take into account an illumination condition (e.g. brightness expressed in cd/m$^2$). Advantageously, the predictive vision model can thus reflect the fact that illumination conditions impact the pupil diameter which in turns impacts the visual performance (the wider the pupil is, the faster the visual performance may deteriorate). This is for instance illustrated by the formula [3] of this disclosure giving visual performance (VP) as a function of the pupil diameter (D_pup).
The predictive vision model may also take into account a visual feature of the target, such as a contrast, or structural feature of the target, such as a spatial frequency or minimum angle of resolution associated to the smallest details of the object to be detected. Advantageously, the predictive vision model can thus reflect the fact that the spatial frequency impacts the depth of focus (the highest the spatial frequencies of the target are, the lower the depth of focus is since the subject is less tolerant). In the predictive vision model, this may be taken into account by making the accommodative gain dependent on the highest spatial frequency of the target.
At the end of step b), the system 10 has computed the accommodation value, the convergence value and the visual performance value.

Step c)

In step c), the accommodation value and/or the convergence value and/or the visual performance value is/are used to assess the suitability.
The system 10 may for instance assess the suitability by comparing at least one of the accommodation value, the convergence value and the visual performance value to a threshold or to a numerical evaluation function.
By way of an example, the suitability may be considered as "high" or "satisfactory" when the visual performance value is above a predetermined value, for instance when the visual acuity is superior to 8/10. Several comparisons (to respective thresholds) may also be performed, for instance one for each one of the accommodation value, the convergence value and the visual performance value, the suitability depending for example on the number of comparisons that are verified.
To assess the suitability, a weighted sum of two or three of the accommodation value, the convergence value and the visual performance value may also be compared to a threshold. Weighting coefficients may be selected based on preferences of the subject which can be obtained directly from questioning the subject or by any statistical analysis or model using data obtained from several subjects.

The system 10 may also assess the suitability by computing at least one visual effort of the subject. The suitability may more particularly be assessed by comparing the at least one visual effort of the subject to a predetermined threshold. The suitability may be considered as "high" or "satisfactory" when the at least one visual effort is below a predetermined value.

The at least one visual effort of the subject is here computed by the system 10 based on a binocular vision effort model stored in its memory 12. This binocular vision effort model here gives a binocular visual effort as the sum of a motor effort and a process effort.

The motor effort Em and the process effort Ep may be expressed as follows for accommodation:

$$Em = km(AR-AX)^2,$$

$$Ep = kp(AS-AR)^2;$$

wherein km and kp are predetermined coefficients, AS is the accommodative demand, AR is the accommodation value, and AX is the reflex response of the accommodation.

The motor effort Em and the process effort Ep may be expressed as follows for convergence:

$$Em = km'(VR-VX)^2,$$

$$Ep = kp'(VS-VR)^2;$$

wherein km' and kp' are predetermined coefficients, VS is the convergence demand, VR is the convergence value, and VX is the reflex response of the convergence.

Optionally, the different efforts may be normalized based on physiological measured values representative of the maximum visual efforts of the subject, such as a physiological threshold where double vision or blur vision appear or disappear (i.e. the point where fusion or single and/or clear vision is recovered).

In an embodiment, the method for assessing the suitability between the corrective optical device 20 and the subject comprises a preliminary step of personalizing the predictive vision model to the subject.

Here, this preliminary step comprises adapting at least one internal parameter of the predictive vision model to the subject. This adaptation may for instance be based on the prescription, the addition, or the age of the subject. This adaptation may also be completed by statistical data.

Preferentially, the system 10 is configured (before calculating the response of the subject) to personalize the predictive vision model based on at least one measured parameter related to the vision of the subject. The at least one measured parameter related to the vision of the subject is for instance: a pupil size, an amplitude of accommodation, an amplitude of convergence, a fusional reserve, a phoria, a dark focus, a tonic accommodation, a blur sensitivity, a visual acuity, a contrast sensitivity, and an interpupillary distance.

By way of an example, the monocular profile of accommodation of the subject, represented in figure 5, may be measured with an automated refractor or accommodometer or aberrometer. The objective accommodation of one eye of the subject is measured while the subject is looking at a visual target (represented by the letter A in figure 5) which moves from far to near in front of the subject. The objective accommodation (in diopters) of the eye may be graphically represented as a function of the target proximity (curve reference C in figure 5, also in diopters).

Internal parameters of the vision prediction model can then be deduced from this curve C. Here, the maximal value of the curve C is the maximal amplitude of objective accommodation ObjAmax of the subject. In figure 5, the maximal amplitude of objective accommodation ObjAmax is approximately 2.25D, obtained for an accommodative demand at a proximity of 3.81 D. The binocular accommodation may then be calculated according to the model by taking into account the interaction between accommodation convergence and the visual performance. The reflex response of the convergence increases the accommodative response without supplementary effort. In the vision prediction model, the dark focus dkF can thus be set according to the formula above (dkF= ObjAmax/3+PRA). The slope of the curve C at half the maximal amplitude of objective accommodation represents the maximum value of the accommodative gain AG.

Other internal parameters of the predictive vision model may also be adjusted such that the response of the subject given by the predictive vision model, in particular the accommodation values, is as close as possible to the measured one (the accommodative demands corresponding to the different positions tested for the visual target), when taking into account the condition of measurements. In other words, the measured monocular profile of accommodation of the subject is a ground truth and the predictive vision model is tuned so that it provides a response as close as possible from this ground truth. The corrective optical device may also include the specific correction used for the particular measurement of the at least one measured parameter related to the vision of the subject, for instance different added prism values on each eye, for phoria and fusional reserves measurements.

When internal parameters of the predictive vision model have been personalized to the subject, preferentially through measurements performed on the subject, the predictive vision model may of course be used to simulate the response of the subject to other visual tasks. For example, a push-down visual task wherein the subject looks at a visual target in front of him with both his eyes, while this target moves from near to far in order to find the position from which the target is seen sharp by the subject. To simulate this experimentation in the virtual optical system, the target distance is gradually increased. At each step, the response of the subject is calculated thanks to the predictive vision model. This operation is repeated for each increment of the target distance until the visual performance reaches a threshold. The position of the object at this level is then considered to be the value of clear vision recovered in the push-down method.

As illustrated in figure 6, the method for assessing the suitability between the corrective optical device 20 and the subject described above is here used within an optical design method, that is to say a method for determining a desired value of the optical feature of a corrective optical device 20.

As shown in figure 6, the method for determining the desired value of the optical feature of the corrective optical device 20 comprises the following steps:

- i) selecting a test value of the optical feature of the corrective optical device 20,
- ii) assessing, based on a method according to claim 15, the suitability between the corrective optical device 20 and the subject when the optical feature of the corrective optical device 20 has the test value,
- iii) determining, based on the suitability and the test value, the desired value of the optical feature of the corrective optical device 20.

Steps i) to iii) are here intended to be repeated if necessary, that is to say iterated, in order to determine the desired value. The general idea is to assess the suitability for the test value, and either to set the desired value as equal to the test value if the suitability is high or, conversely, to modify the test value if the suitability is low (and in this case to repeat steps ii) and iii) to determine the desired value) and to verify if this modified tested value provides a more satisfactory suitability.

The optical feature of the corrective optical device 20 here corresponds to a corrective optical feature of one of the corrective ophthalmic lenses 21. As above-mentioned, the optical feature may be any feature of one of the corrective ophthalmic lenses 21 associated with correcting or improving the vision of the subject such as addition, sphere, cylinder, axis of the cylinder, prism, axis of the prism, near field size, intermediate field size, far field size, progression length, or gradient of progression.

Step i)

In step i), the test value is selected. The test value corresponds to an initial value in the first iteration of steps i) to iii).

The test value may be selected from a prescription previously established for the subject or the current optical correction used by the subject.

An eye-care practitioner can also determine the eyes accommodation and convergence of the subject in relaxed state and deduce the test value based on a simple model of prescription. The test value may also be chosen by the eye-care practitioner based on his experience.

As a variant, the test value may be set to zero or to a value based on the age of the subject.

Step ii)

In step ii), the suitability of the corrective optical device 20 is assessed when its optical feature has the test value. In other words, step ii) corresponds to a particular implementation of steps a) to c) wherein, in the virtual optical system, the corrective optical device 20 numerically complies with the test value.

Here, in step ii), the accommodative demand and/or the convergence demand are determined based on the test value. By way of an example, when the optical feature of the corrective optical device 20 is addition or sphere, the test value may be an optical power value.

At the end of step ii), the suitability of the corrective optical device 20, and thus here the suitability of the test value, is determined.

Step iii)

In step iii), the system 10 is then configured to validate or not the test value based on the suitability determined in step ii).

When the suitability is satisfactory, typically when it is superior to a predetermined threshold, the test value is validated, which means that the desired value is determined as equal to the test value. Steps i) to iii) are thus not repeated.

Conversely, when the suitability is not satisfactory, typically when it is inferior to the predetermined threshold, the test value is not validated, which means that the desired value is determined as not equal to the test value.

In this case, the test value is modified. The test value may for instance be incremented or decremented by a predetermined increment.

After modifying the test value (when the suitability is not satisfactory), steps ii) and iii) are performed again, which is illustrated by a feedback branch in figure 6. If the test value still does not provide a satisfactory suitability, this process is repeated again.

Steps i) to iii) may also be implemented in the form of an optimization process. This optimization process allows determining an optimized test value to which the desired value is considered equal. This optimized test value may correspond to a compromise between the accommodation value, the convergence value and the visual performance value. It may also correspond to the minimal binocular vision efforts.

To determine the optimized test value, any known optimization method can be applied such as gradient descent methods or multi-objective algorithms. As an example, a loss function between the accommodation value, the convergence value and the visual performance value and a numerical evaluation function can be computed by the system 10 and then minimized by modifying the test value.

Several features of the corrective optical device 20 may also be determined at the same time. For instance, it is possible to use a first test value corresponding to the optical power of the corrective optical device 20 and a second test value corresponding to the prismatic deviation power of the corrective optical device 20.

When the suitability is not satisfactory, it is also possible to modify the corrective optical device 20, typically to more complex ophthalmic lenses. Simple unifocal ophthalmic lenses may first be tested by varying its optical power. If the assessed suitability remains low, progressive lenses may be tested.

The optimization may also be performed to get a best compromise between visual performance and design constrains of the corrective optical device 20 such as distortion, far filed size, intermediate filed size, near filed size, gradient of a progressive lens.

As an illustrative example, the method for determining the desired value of the optical feature of the corrective optical device 20 is used to determine the addition that the subject needs at near vision. The method is used to simulate a refraction test. A visual acuity threshold is considered, for instance a near vision visual acuity of 8/10. The specific conditions of the refraction test are specified to the system 10, typically the luminance (for instance 100-200 cd/m$^2$) that will impact the pupil size, the depth of focus and thus visual performance. Then, a desired addition value that gives a visual performance value above the threshold is determined by determining the response of the subject.

As another illustrative example, the addition that the subject needs at near vision could be determine according to the following formula: Add=(S+ + S-)/2 -min((S+ - S-)/2 , k * ObjAA), where: ObjAA the objective amplitude of accommodation, k is a coefficient of effort, S+ and S- are respectively the minimum and maximum powers to add to get a given visual performance.

In other words, S+ is the maximum accommodation point (and thus the minimum provided optical power) and S- the min accommodation point (and thus maximum provided optical power), the addition is the minimum power (S+) giving the tolerated acuity loss, when this point is reachable by accommodation, that is the objective amplitude of accommodation ObjAA (modulated by the effort coefficient k) is higher than (S+ - S-)/2, since (S+ - S-)/2 is representative of the combination of the reachable accommodation amplitude and the depth of focus around the medium accommodative point.

S+ and S- may be calculated thanks to the predictive vision model in conditions of refraction (luminance, target contrast, target spatial frequency, particular lens structure such as progressive lens).

The method for determining the desired value of the optical feature of the corrective optical device 20 may be followed by a step of manufacturing the corrective optical device 20 such that the corrective optical feature complies with the desired value.

The disclosure is not limited to the embodiment described and illustrated and any variant in accordance with the appended claims could be provided.

In particular, the predictive vision model may be used as a monocular predictive vision model. The method for assessing the suitability thus concerns only one eye of the subject. To this end, the convergence gain of the convergence sub-model is set to zero. The method may thus be used for assessing a suitability between one corrective ophthalmic lens and one eye of the subject.

**Claims**

**1.** System (10) for assessing a suitability between a corrective optical device (20) and a subject, the corrective optical device (20) being intended to be worn in front of at least one eye (30) of the subject to improve a vision of the subject, the system (10) being configured to:

- determine an accommodative demand (AS) and a convergence demand (VS) to which the subject is submitted in a virtual optical system, the virtual optical system representing the subject looking at a target (40) through the corrective optical device (20),
- calculate, using a predictive vision model of the subject, a response of the subject to the accommodative demand (AS) and the convergence demand (VS), the response comprising an accommodation value (AR), a convergence value (VR) and a visual performance value (VP), the predictive vision model taking into account interactions between an accommodation sub-model (100) of the predictive vision model, a convergence sub-model (200) of the predictive vision model and a visual performance sub-model (300) of the predictive vision model,
- assess the suitability based on at least one of the accommodation value (AR), the convergence value (VR) and the visual performance value (VP).

2. System (10) according to claim 1, wherein the interactions comprise using an output of one sub-model among the accommodation sub-model (100), the convergence sub-model (200) and the visual performance sub-model (300) as an input of one of the two other sub-models among the accommodation sub-model (100), the convergence sub-model (200) and the visual performance sub-model (300).

3. System (10) according to claim 1 or 2, wherein the predictive vision model is designed such that an output of the visual performance sub-model (300) has a feedback effect on an internal parameter of at least one of the accommodation sub-model (100) and of the convergence sub-model (200).

4. System (10) according to any one of claims 1 to 3, wherein the system (10) is configured to evaluate a depth of focus (DoF) of the subject based on the visual performance value (VP) and to modify an accommodative gain (AG) of the accommodation sub-model (100) as a function of the depth of focus (DoF) of the subject.

5. System (10) according to any one of claims 1 to 4, wherein the system (10) is configured to determine a Panum area (PaRHor) of the subject based on the visual performance value (VP) and to modify a convergence gain (VG) of the convergence sub-model (200) as a function of the Panum area (PaRHor) of the subject.

6. System (10) according to any one of claims 1 to 5, wherein the predictive vision model also takes into account at least one parameter relative to the virtual optical system among an illumination condition and a visual or structural feature of the target (40).

7. System (10) according to any one of claims 1 to 6, wherein the accommodative demand (AS) to which the subject is submitted in the virtual optical system is determined based on a distance (T) between the eye (30) of the subject and the target (40) and on an optical power value (OP) of the corrective optical device (20).

8. System (10) according to any one of claims 1 to 7, wherein the convergence demand (VS) to which the subject is submitted in the virtual optical system is determined based on a distance (T) between the eye (30) of the subject and the target (40) and on a prismatic deviation power value (DP) of the corrective optical device (20).

9. System (10) according to any one of claims 1 to 8, wherein the corrective optical device (20) is **characterized by** its optical surfaces, and wherein at least one of the accommodative demand (AS) to which the subject is submitted in the virtual optical system and the convergence demand (VS) to which the subject is submitted in the virtual optical system is determined by ray tracing based on the optical surfaces of the corrective optical device (20).

10. System (10) according to any one of claims 1 to 9, wherein the system (10) is configured, before calculating the response of the subject, to personalize the predictive vision model based on at least one measured parameter related to the vision of the subject

11. System (10) according to claim 10, wherein the at least one measured parameter related to the vision of the subject is selected in a group consisting of: pupil size, prescription, maximum acuity, maximum objective or subjective amplitude of accommodation, punctum proximum of accommodation, punctum remotum of accommodation, maximum convergence or punctum proximum of convergence, accommodation at rest or dark focus or tonic accommodation, convergence at rest or dark vergence, targeted visual performance for far vision, targeted visual performance for near vision, interpupillary distance or phoria and fusional reserves for far and near distances or any other equivalent optometric measurements.

12. System (10) according to any one of claims 1 to 11, wherein assessing the suitability comprises comparing at least one of the accommodation value (AR), the convergence value (VR) and the visual performance value (VP) to a threshold or to a numerical evaluation function.

13. System (10) according to any one of claims 1 to 12, wherein the system (10) is configured to calculate, based on the virtual optical system and on at least one of the accommodation value (AR), the convergence value (VR) and the visual performance value (VP), at least one visual effort of the subject, and wherein assessing the suitability comprises comparing the at least one visual effort of the subject to a threshold.

14. System (10) for determining a desired value of an optical feature of a corrective optical device (20), the corrective optical device (20) being intended to be worn in front of at least one eye (30) of a subject to improve its vision, the system (10) being configured to:

- select a test value of the optical feature of the corrective optical device (20),
- determine an accommodative demand (AS) and a convergence demand (VS) to which the subject is submitted in a virtual optical system, the virtual optical system representing the subject looking at a target (40) through the corrective optical device (20) when the optical feature of the corrective optical device (20) has the test value,
- calculate, using a predictive vision model of the subject, a response of the subject to the accommodative demand (AS) and the convergence demand (VS), the response comprising an accommodation value (AR), a convergence value (VR) and a visual performance value (VP), the predictive vision model taking into account interactions between an accommodation sub-model (100) of the predictive vision model, a convergence sub-model (200) of the predictive vision model and a visual performance sub-model (300) of the predictive vision model,
- assess a suitability between the corrective optical device (20) and the subject based on at least one of the accommodation value (AR), the convergence value (VR) and the visual performance value (VP),
- determine, based on the suitability and the test value, the desired value of the optical feature of the corrective optical device (20).

15. Method for assessing a suitability between a corrective optical device (20) and a subject, the corrective optical device (20) being intended to be worn in front of at least one eye (30) of the subject to improve its vision, the method comprising:

- determining a virtual optical system representing the subject looking at a target (40) through the corrective optical device (20),
- determining an accommodative demand (AS) and a convergence demand (VS) to which the subject is submitted in the virtual optical system,
- calculating, using a predictive vision model of the subject, a response of the subject to the accommodative demand (AS) and the convergence demand (VS), the response comprising an accommodation value (AR), a convergence value (VR) and a visual performance value (VP), the predictive vision model taking into account interactions between an accommodation sub-model (100) of the predictive vision model, a convergence sub-model (200) of the predictive vision model and a visual performance sub-model (300) of the predictive vision model,
- assessing the suitability based on at least one of the accommodation value (AR), the convergence value (VR) and the visual performance value (VP).

# Fig.1

# Fig.2

# Fig.3

## Fig.4

## Fig.5

## Fig.6

Europäisches
Patentamt

European
Patent Office

Office européen
des brevets

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TW I 797 958 B (GLOBAL OK VISION INC [TW]) 1 April 2023 (2023-04-01) | 1,14,15 | INV. A61B3/00 |
| Y | * abstract * * claims 1-24; figures 2-5 * * paragraphs [0001], [0019] - [0027], [0030] - [0048], [0121] - [0165], [0196] * | 2-13 | A61B3/028 A61B3/04 A61B3/09 A61B3/103 A61B3/08 |
|  | ----- |  |  |
| Y | US 2021/290053 A1 (TRAN TUAN [US] ET AL) 23 September 2021 (2021-09-23) * abstract * * paragraph [0059] - paragraph [0130] * * figures 1-5 * | 2-13 |  |
|  | ----- |  |  |
| A | US 2016/262608 A1 (KRUEGER WESLEY W O [US]) 15 September 2016 (2016-09-15) * abstract * | 1-15 |  |
|  | ----- |  |  |

| | TECHNICAL FIELDS SEARCHED (IPC) |
|---|---|
| | A61B |

The present search report has been drawn up for all claims

| Place of search | Date of completion of the search | Examiner |
|---|---|---|
| Munich | 11 February 2025 | Tommaseo, Giovanni |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.........................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 30 6493

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2025

| Patent document cited in search report | | Publication date | Patent family member(s) | | Publication date |
|---|---|---|---|---|---|
| TW I797958 | B | 01-04-2023 | NONE | | |
| US 2021290053 | A1 | 23-09-2021 | US<br>US<br>WO | 2021290053 A1<br>2024156340 A1<br>2021188584 A1 | 23-09-2021<br>16-05-2024<br>23-09-2021 |
| US 2016262608 | A1 | 15-09-2016 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 4335353 A

**Non-patent literature cited in the description**

- **SHIBATA, T.** ; **KIM, J.** ; **HOFFMAN, D. M.** ; **BANKS, M. S.** The zone of comfort: Predicting visual discomfort with stereo displays. *Journal of Vision*, 2011, vol. 11 (8), 1-29
- **ANDERSON, H. A.** ; **GLASSER, A.** ; **STUEBING, K. K.** ; **MANNY, R. E.** Minus lens stimulated accommodative lag as a function of age. *Optometry and Vision Science*, 2009, vol. 86 (6), 685-694
- **GEORGE K. HUNG**. Quantitative Analysis of the Accommodative Convergence to Accommodation Ratio: Linear and Nonlinear Static Models. *IEEE TRANSACTIONS ON BIOMEDICAL ENGINEERING*, 1997, vol. 44 (4)
- Influence of combined power error and astigmatism on visual acuity. **C. FAUQUIER** ; **T. BONNIN** ; **C. MIEGE** ; **E. ROLAND**. Vision Science and its Applications. Optica Publishing Group, 1995
- **GÓMEZ-PEDRERO JA** ; **ALONSO J.** Phenomenological model of visual acuity. *J Biomed Opt.*, 01 December 2016, vol. 21 (12)
- **ASHRAF, M.** ; **MANTIUK, R. K.** ; **CHAPIRO, A.** ; **WUERGER, S.** astleCSF - A contrast sensitivity function of color, area, spatiotemporal frequency, luminance and eccentricity. *Castle CSF, Journal of Vision*, 2024, vol. 24 (4), 1-38